# EUROPEAN PATENT SPECIFICATION

(11) **EP 1 285 676 B1**
(45) Date of publication and mention of the grant of the patent: **30.03.2011**
(21) Application number: 02102139.9
(22) Date of filing: 14.08.2002
(51) Int. Cl.: A61M 21/00

(54) **Light source for modulation of circadian rhythms**
Lichtquelle zur Modifikation der zirkadianen Rhythmen
Source lumineuse pour la modification des rythmes circadiens

(30) Priority: 14.08.2001 GB 0119735
(43) Date of publication of application: 26.02.2003
(73) Proprietor: OUTSIDE IN (CAMBRIDGE) LIMITED, Cambridge, Cambridgeshire CB3 8UD (GB)
(72) Inventor: Hayes, Stephen Bryce, 34200 Sète (FR)
(74) Representative: Messulam, Alec Moses

(56) References cited:
- WO-A-01/85254
- WO-A-98/51372
- WO-A1-02/20079
- GB-A- 2 333 962
- US-A- 4 444 190
- US-A- 5 824 024

## Description

### Field of the invention

The present invention relates to the use of particular light sources to adjust human body mechanisms.

### Background of the invention

It has long been known that an internal body clock, the body's circadian rhythm, controls the timing and intensity of human physiological functions such as eating, sleeping, etc. While the circadian effect is innate, it is normally entrained to the daylight/night-time cycle and can be modulated by timed exposure to bright light. For example, WO 02/20079 A1, published after the priority date of this patent, describes a method for controlling the alertness of a human subject and a light source for use in this method, which method comprises exposure of the subject during an exposure period to suitable light radiation without substantially influencing the phase of a melatonin cycle.

It is also known that light can be used in the treatment of certain conditions. US-A-4,444,190 discloses a device for phototherapeutic treatment of hyperbilirubinemia. The latter patent teaches that it is beneficial to concentrate the radiation in the range of 460 to 480nm.

GB 2,333,962 teaches modulating the circadian rhythm by exposing a non-ocular region of the body to light. For such extra-ocular stimulation WO98/51372 teaches that light in the blue-green range of the spectrum (455-540nm) is preferred. The preamble of claim 1 is e.g. disclosed in US 5,824,024 teaching simulation of daylight (white light) in accordance with a predetermined sequence.

### Object of the invention

The present invention is predicated on the discovery that particular wavebands of visible light are more effective in affecting or modulating circadian effects and other photo-dependent human body mechanisms and seeks to provide light sources that make practical use of this discovery.

### Summary of the invention

According to the present invention there is provided a light source for producing light containing spectral components having wavelengths distributed throughout the visible spectrum so as to be perceived by the human eye to be substantially white light and adapted to modulate photo-dependent human body mechanisms; wherein the light source is combined with controlling or switching means for operating the light source in accordance with a predetermined programme, wherein said controlling or switching means include timing means capable of being programmed to provide light at appropriate times and in appropriate duration/intensity combinations in accordance with a predetermined sequence; characterised in that the energy of the light emitted by the source within an anomalous waveband from 459 to 464nm is significantly greater than the energy emitted by the source in any other waveband of equal width.

The advantage of the light source of the present invention is that it emits what passes for white light or natural daylight, which is generally more comfortable on the eyes and does not interfere with the perception of colour. Nevertheless the important waveband around 460nm, which is believed to be the vital waveband for affecting the circadian rhythm, is modified from normal white light to enhance or suppress stimulation of the body's circadian rhythm.

According to the invention, the energy of the light emitted within the anomalous waveband is significantly greater than the energy emitted by the source in any other waveband of equal width. Such a light source can be used to assist in resetting the body clock and has an enhanced effect on the circadian rhythm as compared with a conventional source of white light of the same light output power.

The energy of the light emitted within the anomalous waveband may be significantly less that the energy emitted by the source in any other waveband of equal width. In this case, the light source can switched on while minimising the disturbance to the circadian rhythm. Patients in intensive care may need to be illuminated during the night so that they may be monitored. By using a light source from which emissions in the waveband around 460nm are suppressed, it is possible to minimise the disturbance to the patients body clock when switching the light on.

The waveband within which enhancement of light energy is most effective is 440 to 480nm. However, it is known that excessive exposure to blue light may induce short term visual deterioration or even more permanent damage. In order to reduce so-called "blue light hazard", the waveband of the light source in which light emission is enhanced is preferably concentrated in the range of 460 to 480nm. A particularly effective waveband is 459 to 464nm.

It will be appreciated that these wavelengths cannot be considered as rigid limits, since there is rarely a sharp cut-off in the spectral distribution.

Depending on the intensity and duration of exposure, it may be advisable to filter or weight the lower wavelengths to reduce blue light hazard, in accordance with the Commission International de l'Eclairage consensus formula or other published standards.

The conventional wisdom is that artificial light levels in excess of 2500 lux, normally up to 10000 lux, are required to achieve a circadian effect. Using the present invention, it is possible to affect or modulate circadian effects and other photo-dependent human body mechanisms while using light sources where the overall level of illumination is substantially below 1200 lux. For example, significant circadian effects may be achieved or generated by timed application of 100x10¹² photons.cm².s⁻¹ in the 459 to 464nm waveband, corresponding to greater than 90% reduction in light level.

Preferred light energy for enhancing modulation of the circadian rhythm in the selected wavebands is 20x10¹² to 100x10¹² photons.cm⁻².s⁻¹.

A particular application of the present invention is in alleviation of the temporal and physiological disorientation associated with rapid transit across several time zones ("jet lag") or consequent upon a work or activity pattern outside normal daytime hours. In such cases, the body's circadian rhythms are adjusted by exposure to sufficient light in the selected waveband at appropriate times and in appropriate duration/intensity combinations, in accordance with a predetermined programme. Another application of the present invention is in the alleviation of sleep disorders.

Many conventional phototherapy methods comprise alternation of light and dark cycles according to a predetermined programme. A light source in which the 460nm waveband is suppressed can be used to provide illumination during the "dark" cycle of such a programme. Conveniently, the light energy emitted by such a source at wavelengths below 480nm or at least in the range between 440nm and 480nm does not exceed 1x10¹² photons.cm⁻².s⁻¹.

In these and other cases, the pre-determined light application and/or avoidance cycle may be modulated according to a measured physiological state of the subject recipient, in particular the timing or occurrence of body minimum temperature or variations in resting blood pressure. Another physiological state that may be measured is the level of circulating melatonin and, in particular, the established phenomenon of "dim light melatonin onset". The predetermined programme, and particularly the timing of selected cycles, is varied in a manner directly or indirectly connected with variations in such physiological states.

The above applications are believed to involve adjustment or synchronisation of circadian effects, but the present invention is equally applicable to other photo-dependent body mechanisms not directly linked to the circadian (daily) cycle. For example, exposure to cycles of bright light is commonly used to reduce or alleviate depressive or quasi-depressive syndromes and affective disorders, and particularly Seasonal Affective Disorder (S.A.D.). Phototherapy may help to alleviate some of the effects of degenerative conditions and dementias such as Alzheimer's.

The required distribution of the radiation within the visible spectrum may be achieved by combining light from different light sources having different spectral distributions and/or by filtering light from one or more sources through suitable filters such as dichroic filters.

The individual sources used to generate the light may be one or more chosen from the group comprising fluorescent tubes where the tube phosphors are suitably selected to emit or not to emit light in the selected waveband, integrated circuit or semiconductor emitters such as a light emitting diodes, and incandescent bulbs, especially halogen or krypton bulbs.

The light source may be stand-alone or incorporated into a lamp or luminaire.

For many applications, a light source according to the present invention may be substituted in existing products such as light boxes, lamps or visors. An advantage of incorporating a light source according to the present invention in a luminaire, is that the luminaire may additionally include conventional light sources the light from which is differently directed. In this way, light from the source of the invention may be directed towards an intended recipient while the general illumination from the conventional light source is directed elsewhere at a different, for example lower, intensity.

Instead of being free-standing, a light source according to the present invention may be mounted on a human body, for example incorporated into head-worn devices such as goggles or a visor.

The output of a light source according to the present invention may be directed extraocularly and especially to blood vessels in or subjacent to the skin. Patent Application No. GB 2 333 962 A dated 4 February 1999 describes and claims a method and means of adjusting the circadian rhythms of a human body by the application of light, extraocularly, to one or more areas of the body surface, at appropriate times and for appropriate periods, in accordance with a predeterminable programme. A light source according to the present invention may be used in implementing the invention in that Patent Application. As described therein, the light source may be associated with positioning means to locate the source over blood vessels in or subjacent to the skin.

A light source of the present invention may be switched or controlled in known manner. In particular, the output waveband and/or intensity may be varied, either manually or in accordance with a predetermined programme. Similarly, the light source may be switched on and off at particular times and the duration, intensity and/or waveband of illumination varied in accordance with a predetermined sequence. As is already known, phototherapy programmes may employ cycles of any chosen period and are not limited to 24 hours.

An advantage of the combination of a light source according to the present invention with other lamps, as discussed above, is the possibility of the two systems being switched or controlled independently of each other. A luminaire incorporating at least one additional light source may direct the light output from a light source or sources according to the present invention towards an intended recipient while the additional light source or sources provide general illumination of the whole or part of the surroundings at times, intensity or wavelength independent of said directed output. For example, a single luminaire may direct sufficient light energy with the desired spectral distribution directly to a recipient while concurrently illuminating the surroundings or a particular object such as a book with light or combinations of lights of different visual appearance and/or intensity.

By use of appropriate combinations of controllers or programmers, a phototherapy programme may be provided concurrently with independent variation of overall or background illumination, for example to simulate the natural variations in light levels and/or colour temperature over the course of a day.

As discussed above, there are situations where it is desirable or necessary to exclude light within the 460nm waveband. For example, it is believed that sleep cycles are affected by levels of circulating melatonin and that melatonin production is suppressed by light of sufficient intensity. A lamp or luminaire may be adapted to provide sufficient white light illumination while avoiding exposure of a recipient to light of a wavelength shorter than 480nm, or at least light in the range from 440nm to 480nm, when such exposure is inappropriate. Night staff in a hospital could see to work without disturbing patients' sleep cycles.

Shift workers or air pilots may continue to operate while avoiding selected wavelengths at inappropriate times in a particular light/dark cycle.

In association with appropriate time sequences or controllers, such results can be achieved by employing a light source substantially excluding light output at wavelengths shorter than 480nm (or at least wavelengths between 440nm and 480nm) or by filtering such wavelengths from conventional light sources or natural daylight.

It should be mentioned that the research on which the present invention is based analysed the spectral distribution of the light impacting the retina of the eye. Having regard to the fact that the transmission of light may be affected by the fluids within the eye, the centre of the anomalous waveband of the light emitted by the light source may need to be shifted slightly from 460nm to optimise ocular modulation of circadian rhythms.

## Claims

1. A light source for producing light containing spectral components having wavelengths distributed throughout the visible spectrum so as to be perceived by the human eye to be substantially white light and adapted to modulate photo-dependent human body mechanisms; wherein the light source is combined with controlling or switching means for operating the light source in accordance with a predetermined programme, wherein said controlling or switching means include timing means capable of being programmed to provide light at appropriate times and in appropriate duration/intensity combinations in accordance with a predetermined sequence; **characterised in that** the energy of the light emitted by the source within an anomalous waveband from 459 to 464nm is significantly greater than the energy emitted by the source in any other waveband of equal width.

2. A light source as claimed in Claim 1, which includes one or more light sources selected from the group comprising fluorescent tubes wherein the tube phosphors are suitably selected to emit light in the selected waveband; integrated circuit or semiconductor emitters such as a light emitting diodes; and incandescent bulbs, especially halogen or krypton, bulbs.

3. A light source as claimed in Claims 1 or 2, further comprising an optical filter.

4. A light source as claimed in claim 3, wherein the filter has a pass band that coincides with the anomalous waveband.

5. A light source as claimed in any preceding claim, having means for mounting the light source on a human body.

6. A light source as claimed in any one of the preceding claims adapted to direct light extraocularly to one or more areas of the body surface.

7. A light source as claimed in any one of the preceding claims, in combination with means for monitoring a physiological state of an intended recipient, wherein the controlling means is adapted to alter at least one parameter of the light source or the application cycle in accordance with such physiological state.

8. A light source as claimed in claim 7, wherein said physiological state is the body minimum temperature or the dim light melatonin onset.

9. A luminaire incorporating a first light source as claimed in any preceding claim for illuminating an intended recipient and further comprising a differently directed additional light source to provide ambient illumination.

10. A luminaire as claimed in claim 9, wherein the first light source and the additional light source are switchable or controllable independently of each other.

## Patentansprüche

1. Eine Lichtquelle zur Erzeugung von Licht, das spektrale Komponenten enthält, deren Wellenlängen sich über das gesamte sichtbare Spektrum erstrecken, so dass sie vom menschlichen Auge mehr oder weniger als Weißlicht wahrgenommen und adaptiert wird, um die lichtabhängigen Mechanismen des menschlichen Körpers zu modulieren, wobei die Lichtquelle mit Steuer- oder Schalteinrichtungen kombiniert wird, um die Lichtquelle entsprechend eines vorbestimmten Programms zu operieren, wobei zur genannten Steuer- oder Schalteinrichtung Zeiteinstellmöglichkeiten gehören, die so programmiert werden können, um Licht zu entsprechenden Zeiten und in entsprechenden Dauer-/Intensitätskombinationen entsprechend einer vorbestimmten Folge zu liefern,
**dadurch gekennzeichnet, dass** die von der Lichtquelle von innerhalb eines normalen Wellenbands 459 bis 464 nm emittierte Energie bedeutend größer ist als die Energie, die von der Quelle in einem anderen Wellenband gleicher Breite emittiert wird.

2. Eine Lichtquelle entsprechend Anspruch 1, zu der eine oder mehrere Lichtquellen gehören, die aus der Gruppe bestehend aus folgenden ausgewählt werden, nämlich Leuchtstoffröhren, bei denen die Phosphore entsprechend ausgewählt werden, um Licht im gewählten Wellenband zu emittieren, integrierte Schaltkreise oder Halbleiteremitter, wie z.B. Leuchtdioden, und Glühbirnen, insbesondere Halogen- oder Krypton-Glühbirnen.

3. Eine Lichtquelle entsprechend Anspruch 1 oder 2, zu der zudem ein optischer filter gehört.

4. Eine Lichtquelle entsprechend Anspruch 3, wobei der Filter eine Durchlassbreite hat, die dem anomalen Wellenband entspricht.

5. Eine Lichtquelle entsprechend einem der vorhergehenden Ansprüche, zu der die Möglichkeit gehört, die Lichtquelle am menschlichen Körper zu befestigen.

6. Eine Lichtquelle entsprechend einem der vorhergehenden Ansprüche, die so adaptiert ist, dass sie Licht extraokulär auf einen oder mehrere Bereiche der Körperoberfläche richtet.

7. Eine Lichtquelle entsprechend einem der vorhergehenden Ansprüche in Kombination mit Möglichkeiten zur Überwachung eines physiologischen Zustands eines beabsichtigten Empfängers, wobei die Steuereinrichtung so adaptiert ist, dass sie zumindest einen Parameter der Lichtquelle oder des Applikationszyklus entsprechend dieses physiologischen Zustands ändert.

8. Eine Lichtquelle entsprechend Anspruch 7, wobei der genannte physiologische Zustand die Mindestkörpertemperatur oder der Beginn der Melatoninsekretion (Dim Light Melatonin Onset) ist.

9. Eine Leuchte, in die eine Lichtquelle entsprechend einem der vorhergehenden Ansprüche inkorporiert ist, zur Illuminierung eines beabsichtigten Empfängers, zu der zudem eine anders ausgerichtete zusätzliche Lichtquelle gehört, um Umgebungsbeleuchtung zu liefern.

10. Eine Leuchte entsprechend Anspruch 9, wobei die erste Lichtquelle und die zusätzliche Lichtquelle unabhängig voneinander geschaltet oder gesteuert werden können.

## Revendications

1. Source lumineuse pour la production de composants spectraux contenant de la lumière possédant des longueurs d'ondes réparties dans l'ensemble du spectre visible de manière à être perçue par l'oeil humain comme étant sensiblement une lumière blanche et adaptée à moduler des mécanismes photo-dépendants du corps humain ; dans laquelle ;
la source lumineuse est combinée à des moyens de commande ou de commutation pour le fonctionnement de la source lumineuse selon un programme prédéterminé, dans laquelle lesdits moyens de commande ou de commutation comportent des moyens de temporisation aptes à être programmés de manière à fournir de la lumière à des périodes appropriées et selon des combinaisons de durée/intensité appropriées conformément à une séquence prédéterminée ;
**caractérisée en ce que** l'énergie de la lumière émise par la source au sein d'une bande d'ondes anormale allant de 459 à 464 nm est significativement supérieure à l'énergie émise par la source dans toute autre bande d'ondes de largeur égale.

2. Source lumineuse selon le procédé de la Revendication 1, qui comprend une ou plusieurs sources lumineuses choisies dans le groupe comprenant des tubes fluorescents dans lesquels les phosphores des tubes sont adéquatement sélectionnés pour émettre de la lumière dans la bande d'ondes sélectionnée ; un circuit intégré ou des émetteurs de semiconducteurs tels que des diodes électroluminescentes ; et des ampoules incandescentes, en particulier des ampoules à halogène ou au krypton.

3. Source lumineuse selon le procédé de la Revendication 1 ou 2, comprenant en outre un filtre optique.

4. Source lumineuse selon la Revendication 3, dans laquelle le filtre possède une bande passante qui coïncide avec la bande d'ondes anormale.

5. Source lumineuse selon le procédé de l'une quelconque des revendications précédentes, possédant des moyens pour le montage de la source lumineuse sur un corps humain.

6. Source lumineuse selon le procédé de l'une quelconque des revendications précédentes adaptée pour diriger la lumière de façon extraoculaire vers une ou plusieurs zones de la surface corporelle.

7. Source lumineuse selon le procédé de l'une quelconque des revendications précédentes, utilisée en association à des moyens de surveillance d'un état physiologique d'un receveur prévu, dans laquelle le moyen de commande est adapté pour modifier au moins un paramètre de la source lumineuse ou le cycle d'application en fonction dudit état physiologique.

8. Source lumineuse selon le procédé de la Revendication 7, dans lequel ledit état physiologique est la température corporelle minimale ou le déclenchement de la mélatonine sous lumière faible.

9. Luminaire incorporant une première source lumineuse selon le procédé de l'une quelconque des revendications précédentes pour l'illumination d'un receveur prévu et comprenant en outre une source de lumière supplémentaire dirigée de façon différente afin de fournir une illumination ambiante.

10. Luminaire selon le procédé de la revendication 9, dans lequel la première source de lumière et la source de lumière additionnelle sont commutables ou contrôlable indépendamment l'une de l'autre.
